# EUROPEAN PATENT APPLICATION

(11) **EP 3 196 666 A1**
(43) Date of publication of application: **26.07.2017**
(21) Application number: 16152035.8
(22) Date of filing: 20.01.2016
(51) Int. Cl.: G01R 33/48, G01R 33/56, G01R 33/561, G01R 33/565, A61B 5/055

(54) **MAGNETIC RESONANCE IMAGING METHOD FOR DETECTING NEURONAL ACTIVITY IN A SUBJECT'S BRAIN**

(71) Applicant: Universität Bern, 3012 Bern (CH)
(72) Inventor: The designation of the inventor has not yet been filed
(74) Representative: Schmauder & Partner AG Patent- & Markenanwälte VSP

(57) **Abstract**

A magnetic resonance (MR) imaging method for detecting neuronal activity in a subject's brain comprises performing a first MR imaging sequence part a) comprising a preparatory spin-locking pulse sequence part followed by an acquisition pulse sequence part, followed by a second MR imaging sequence part b) comprising the same acquisition pulse sequence part, whereafter steps a) and b) are repeated for a predetermined number of instances, each time using a different initial spin-lock phase. In order to detect neuronal activity in a subject's brain the MR imaging method is carried out with the subject's brain positioned in the MR imaging system; difference MR data are obtained by subtracting said second MR data from said first MR data, wherein zones of comparatively high brightness are indicative of fast oscillating neuronal currents.

## Description

### Field of the Invention

The present invention generally relates to a magnetic resonance (MR) imaging method, particularly for detecting neuronal activity in a subject's brain. Moreover, the invention relates to a method of detecting neuronal activity in a subject's brain, and to a computer program product for causing a computer system to control the execution of the method.

### Background of the Invention

Neuronal current imaging (NCI) refers to a number of magnetic resonance (MR) methods that measure small local perturbations of the MR signal phase and / or amplitude induced by weak electromagnetic fields that accompany neuronal signaling (1-3).

For clinical purposes, NCI developments are straightforwardly directed towards the investigation of interictal epileptic discharges (IED) in epilepsy. This is motivated by the fact that (i) neuronal currents accompanying IED should be strong enough to be detected using standard clinical B0 field strengths, (ii) NCI offers the potential to supersede metabolic imaging techniques as PET and EEG/fMRI due to their direction to electric fields and comparably high spatiotemporal resolution and (iii) an MR-based technique that integrates knowledge about the structural epileptogenic lesion and markers of their epileptogenicity in a single examination is still lacking.

Accordingly, it is an object of the present invention to provide an improved MR imaging method, particularly for detecting neuronal activity in a subject's brain.

Further objects of the invention are a method of detecting neuronal activity in a subject's brain, particularly in relation to epileptic events, and a computer program product for execution of the detection method.

### Summary of the Invention

According to one aspect of the invention, there is provided a magnetic resonance (MR) imaging method, particularly for detecting neuronal activity in a subject's brain, the method comprising:
a) performing a first MR imaging sequence part with a MR imaging system having a static magnetic field in z-direction, said first sequence part comprising:
   - a preparatory spin-locking pulse sequence part comprising an initial spin-tipping pulse followed by a spin-lock pulse followed by a final spin-tipping pulse, followed by spoiler gradient pulses for removing any remaining transverse magnetization;
      wherein said initial spin-tipping pulse is a 90° pulse with an initial phase ϕ_{1SL} in the rotating system and wherein said final spin-tipping pulse is a 90° pulse with a final phase ϕ_{1SL} + 180°;
      wherein said spin-lock pulse consists of a rectangular pulse having an amplitude B_{1lock} and a spin-lock pulse duration TSL and consisting of a first pulse portion with a first phase ϕ_{1SL} + 90° followed by a second pulse portion with a second phase ϕ_{1SL} - 90° or, vice versa, consisting of a first pulse portion with a first phase ϕ_{1SL} - 90° followed by a second pulse portion with a second phase ϕ_{1SL} + 90°, said spin-lock pulse having an amplitude B_{1lock} corresponding to a ¹H-Larmor frequency of 100 to 200 Hz;
   - an acquisition pulse sequence part to acquire first MR data, wherein said acquisition pulse sequence part is an echo planar imaging sequence;
   said step a) being followed or preceded by step b)
b) performing a second MR imaging sequence part with said MR imaging system, said second sequence part consisting of said acquisition pulse sequence part to acquire second MR data;
c) repeating steps a) and b) for a predetermined number of instances, wherein for each instance the initial spin-lock phase ϕ_{1SL} is increased, with respect to the preceding instance, by a predetermined phase increment Δϕ_{1SL}, thereby obtaining multiple sets of first and second MR data;
d) averaging said first MR data and said second MR data to obtain averaged first MR data and averaged second MR data, respectively;
e) obtaining difference MR data by subtracting said averaged second MR data from said averaged first MR data; and
f) generating at least one slice image from said difference MR data, wherein zones of comparatively high brightness are indicative of fast oscillating neuronal currents with a frequency in the range of 100 to 200 Hz.

The MR imaging system is of a generally known type and comprises means for generating a static, i.e. stationary homogeneous magnetic field in z-direction, means for exciting RF pulses, means for generating magnetic field gradients, means for detecting and sampling magnetic resonance signals obtained with said means in an object or subject which is situated in the homogeneous field, control means for controlling said means, image reconstruction means, and controller means programmed to run any predetermined MR imaging sequences.

In the present context, the term "MR imaging sequence" shall be understood as a temporal sequence of steps as generally known in the field of MR imaging, including RF pulses of various shapes and gradient field pulses. Although the term "sequence" is often understood as meaning the entirety of individual steps needed to carry out a complete measurement or image acquisition, in some situations it is also used to denote just a subset of steps that are repeated either identically or with some modification for a plurality of times, so that it will be more appropriate to speak of a "sequence portion" or "sequence part". In the present context, the term "sequence part" will be used to denote any specific set of steps that are carried out as part of what could be understood as the "over-all sequence" of steps needed to complete the claimed method.

The method of the present invention relies on an extension of a basically known method - i.e. the spin-locking (SL) technique - to NCI (4,5), further referred to as "phase-cycled stimulus-induced rotary saturation approach with spin-lock preparation" (pc-SIRS). With this method, we obtain a spatially encoded signal using changes in MR signal magnitude that reflect local electromagnetic fields generated by fast oscillations during IEDs (6). It was surprisingly found that non-hemodynamic resonant saturation effects arise from perturbations of the magnetic field generated by fast oscillating neuronal currents that are signatures of epileptogenic brain tissue as determined by invasive EEG recordings and cortical potential distributions of surface EEGs. To prove that these effects are related to IEDs, the observed effects should be cancelled after successful epilepsy surgery in a subset of patients. Therefore, we investigated whether non-hemodynamic resonant saturation effects can be detected in focal epilepsy patients using pc-SIRS, and whether they colocalize with seizure onset zone (SOZ) and surface interictal epileptiform discharges (IED).

The method of the present invention is based on an overall sequence comprising two subsequences called here "first MR imaging sequence part" and "second MR imaging sequence part" leading to the acquisition of first MR data and second MR data, respectively. In simple terms, the first MR imaging sequence part comprises a preparatory pulse sequence part followed by an acquisition pulse sequence part, whereas the second MR imaging sequence part merely comprises the acquisition pulse sequence part.

The first MR imaging sequence part corresponds to a pulse sequence for rapid in vivo spin-locked MRI that has been previously described by Borthakur et al., 2006. It generally comprises a preparatory spin-locking pulse sequence part and an acquisition pulse sequence part.

The preparatory spin-locking pulse sequence part comprises an initial spin-tipping pulse configured as a 90° pulse in y direction, which flips the overall magnetization into the xyplane. Thereafter, a spin-lock pulse configured as a rectangular pulse in x direction and having an amplitude B_{1lock}, a spin-lock pulse duration TSL and an initial spin-lock phase ϕ_{1SL} is applied. As explained in more detail further below, the Larmor frequency during the spin-lock pulse is shifted from RF to a substantially lower frequency and thereby allows for sensitive acquisition of signals with components in a low frequency range. After the spin-locking period, a final spin-tipping pulse configured as a 90° pulse in -y direction is applied to return the overall magnetization in z direction. It will be understood that equivalent results could be obtained by switching the sign of the two 90° pulses. In order to remove any transverse magnetization, a series of spoiler gradient pulses are applied after the final spin-tipping pulse. The spin-lock pulse consists of two phase-alternating halves, i.e. the second half is phase-shifted by ±90° from the phase of the first half, which allows reducing the undesirable effects of B1 field inhomogeneity.

The acquisition pulse sequence part is an echo planar imaging (EPI) sequence. The same acquisition pulse sequence part is used in the first MR imaging sequence part a) and in the second MR imaging sequence part b).

The above mentioned first and second MR imaging sequence parts form the building blocks of an "on-off-spin-locking scheme". This scheme is repeated for a predetermined number of instances, whereby the initial spin-lock phase ϕ_{1SL} is increased by a predetermined phase increment Δϕ_{1SL} so as to obtain multiple sets of first and second MR data. For the avoidance of doubt, it shall be understood that Δϕ_{1SL} could be positive or negative.

The entire data acquisition process just described can itself be repeated for signal averaging purposes so as to obtain the required data quality.

The multiple sets of first and second MR data are generally stored for subsequent data processing, particularly for the purpose of image reconstruction.

Advantageous embodiments of the invention are defined in the dependent claims and/or are described herein below.

According to the invention, the acquisition pulse sequence part is an echo planar imaging sequence, for which various embodiments are known.

According to an advantageous embodiment (claim 2), the echo planar imaging sequence is a gradient echo echo planar imaging sequence (GRE). This name is related to the fact that in GRE sequences the 180° echo-forming pulse is omitted and the magnetization is refocused solely by appropriately configured gradient pulses.

According to an advantageous embodiment (claim 2), the spin-lock pulse has an amplitude B_{1lock} corresponding to a Larmor frequency of 100 to 200 Hz, preferably about 120 Hz. This has been found to be particularly suitable for the detection and concurrent imaging of neuronal currents in a subject's brain.

According to a further embodiment (claim 3), the spin-lock pulse duration TSL is 50 to 200 ms, preferably about 100 ms. This has been found to be advantageous in terms of signal quality and acquisition time.

According to a further embodiment (claim 4), the phase increment Δϕ_{1SL} is 40° to 60°, preferably about 50°. This so-called "phase cycling" allows for detection of dipoles of different orientations.

In contrast to other methods, most notably the so-called BOLD (Blood Oxygenation Level Dependent) method, the detection method of the present invention provides a result that is directly related to certain neuronal current components in a subject's brain. It will thus be denoted here as a method of neuronal current imaging and henceforth abbreviated as "NCI". As will be discussed further below, if neuronal events in the subject's brain lead to magnetic pulses that are collinear with the spin-locking axes, a resonance condition is fulfilled and a pre-saturation of magnetization occurs during the spin-locking pulse sequence part. Thereafter, the signal acquired by means of the acquisition pulse sequence part will be lower than the signal acquired by means of the same acquisition pulse sequence part without preceding spin-lock. When epileptic events lead to magnetic pulses having a modulation frequency clearly higher than the spin-lock frequency, the resonance condition is not fulfilled and thus the spin-locked related signal will be similar to that without spin-lock.

The term "fast oscillating neuronal currents" refers to frequency components in the acoustic range, particularly in the range of 100 to 200 Hz, preferably about 120 Hz.

According to one embodiment (claim 5), the image is represented as a z-score map obtained by a raw image reconstructed from said difference MR data. The use of z-score maps is known in MRI, but it turns out to be particularly helpful in improving the quality of images obtained according to the present invention.

According to a further embodiment (claim 6), the z-score map includes a threshold of 50 to 70%, preferably about 60%. The latter threshold value has been found suitable for acquiring NCI related images over a broad range of experimental conditions.

According to a further aspect of the invention, there is provided a computer program product comprising program code means stored on a computer readable medium for causing a computer system to control the execution of the method of detecting neuronal activity in a subject's brain when the computer program product is run on the computer system. The computer readable medium on which said program code means are stored are any suitable data storage means such as transportable storage media, e.g. DVD or memory sticks, or storage media permanently installed in a computer or processor linked to a MRI apparatus, or remote storage media accessible via a computer network or cloud.

### Brief description of the drawings

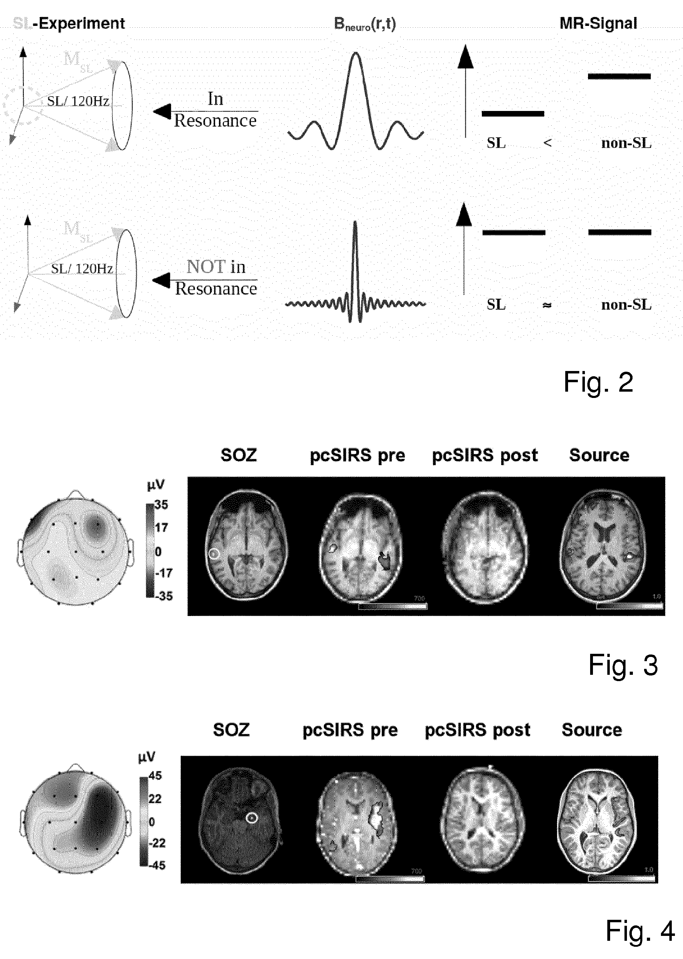

The above mentioned and other features and objects of this invention and the manner of achieving them will become more apparent and this invention itself will be better understood by reference to the following description of various embodiments of this invention taken in conjunction with the accompanying drawings, wherein are shown:
**Figure 1****:**
   The pc-SIRS sequence based on a spin-locking preparation and echo-planar readout (ADC: analog digital converter). For further details see text.
**Figure 2****:**
   Relevant principles of the spin-locking (SL) based pc-SIRS technique. Blue axis: direction Bₙₑᵤᵣₒ, green axis: direction B_{Scanner/SL}, dashed green circle symbolizes rotation of spin-locked magnetization M_{SL} around the blue axis in case of on-resonance. A typical spin-locking (pc-SIRS) experiment consists of
   1) Spin-Locking with Scanner Pulse B_{Scanner/SL} (rectangular pulse with length TSL) → locked magnetization M_{SL}
   2) Bₙₑᵤᵣₒ (excitation or saturation field due to oscillating neuronal currents J(r,t)) on-resonant with locked magnetization M_{SL} at frequency γB_{Scanner/SL} → rotation of M_{SL} → excitation resp. saturation effect.
   For the relation of Bₙₑᵤᵣₒ with the intracranial currents J(r,t), see supplementary materials. (The analogy with other references (e.g. Witzel et al.) is as follows: Bₙₑᵤᵣₒ ~ B_{rotarysat}, B_{Scanner/SL} ∼ B_{1,lock})
**Figure 3****:**
   Exemplary results in a patient with left temporal lobe epilepsy (case P05). The left most image shows a scalp voltage map of interictal epileptiform discharges (IED) with a the maximum negativity over anterior fronto-temporal regions. The second image shows the location of the seizure onset zone (SOZ). The red dots indicate positions of non-SOZ intracranial electrodes, the green encircled dot the electrode with the first seizure signals, as determined by visual analysis. The second and third panels shows the distribution of pc-SIRS effects before surgery (pre), and their resolution after surgery (post). The final panel shows a map of the source reconstruction, based on the IED map of the first panel. All images are in neurological convention (left in the image corresponds to the left hemisphere).
**Figure 4**
   Exemplary results in a patient with right mesial temporal lobe epilepsy (case P07). The sequence of panels is the same as in figure 3.
**Figure 5****:** pc-SIRS sequence signal simulation: no stimulated or indirect echoes could be detected (Red box: main echo).

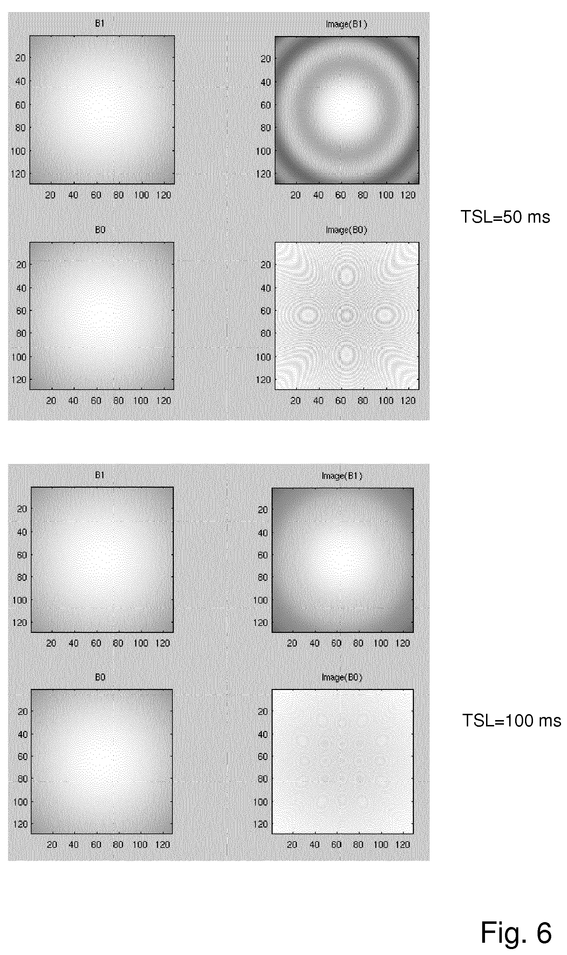

**Figure 6****:** Simulation of B0/1 effects depending on the length TSL of the spin locking pulse based on the Bloch equations, assuming initial Gaussian B0/1 distributions. Ringing artifacts on the Mz maps (Image(BO/1)) maps are less pronounced in case of TSL=100ms than in case of TSL=50ms.

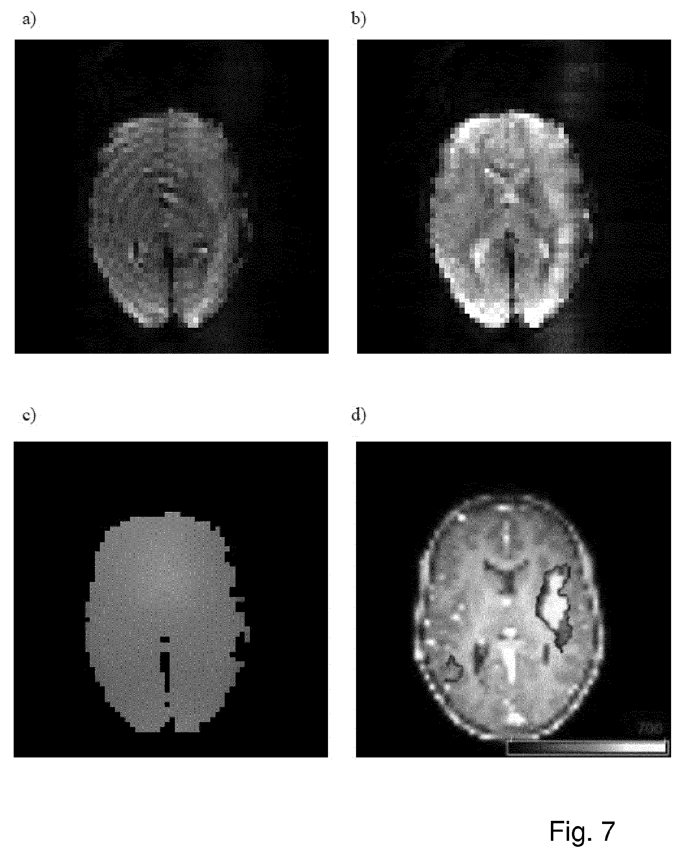

**Figure 7****:** The pc-SIRS effects of , as exemplarily shown here for P 07, (see figure 5) do not correlate with T2* resp. phase effects. The first row indicates pc-SIRS magnitude images of the SL (a) and non-SL (b) acquisition of the exemplary slice, the second row pc-SIRS phase difference (SL minus non-SL acquisition) (c) and pc-SIRS magnitude + z-scores (overlay) (d).
**Figure 8****:** 10/20 electrode positions used for IED mapping
**Figure 9****:** Results of EEG and imaging analysis in all patients.
   Column (A): Scalp potential of interictal epileptiform discharges. Column (B): Localization of the seizure onset zone on MR-CT fusion images (white circles). Column (C): Clusters of pc-SIRS effects (z-scores) ahead of resective surgery Column (D): Clusters of pc-SIRS effects (z-scores) after resective surgery (P02, P03, P05,P06, P07) Column (E): Results of EEG source reconstruction. Clusters indicate the maximum a posteriori estimates of the source localizations, taking into account the pc-SIRS clusters of colum(C) as prior information. The source estimation in the low density surface EEG yielded comparable localization if a pc-SIRS-informed source reconstruction was applied.

### Detailed description of the invention

### Experimental Study: MR-imaging of non-hemodynamic effects in focal epilepsies using a phase-cycled stimulus-induced rotary saturation approach with spin-lock preparation (pc-SIRS).

### Materials and Methods

### Subjects

The study was approved by the local ethical committee and patients gave written informed consent before enrollment. We prospectively recruited consecutive patients with focal epilepsies that underwent presurgical evaluation at our hospital between March 2013 and September 2014. Inclusion criteria were: (1) confirmed epilepsy, based on clinical history, scalp EEG (sEEG), and routine clinical MR imaging, (2) drug resistance according to the consensus criteria of the International League Against Epilepsy (ILAE), (3) sEEG with interictal epileptiform discharges, (4) intracranial EEG (iEEG) recordings with at least two clinically and electroencephalographically identical seizures, (5) EEG measurements within 14 days of MR image acquisition. Exclusion criteria were: (1) no consent, (2) metallic implants, claustrophobia or other contraindications for MR imaging, (3) multifocal or no seizures on iEEG. Of 9 recruited patients, 1 was excluded due to multifocal seizures. In all 8 remaining patients, one run of the proposed SL-EPI sequence was acquired during a standard clinical MR imaging session performed before iEEG implantation (6). For each patient, post-implantation computed tomography (CT) scans were also available. In 5 patients, we acquired a second SL-EPI run after resective surgery. Median time between first and second run was 178 days (range 7-218 days). Clinical data are summarized in Table 1.

### PC-SIRS and MR Imaging Protocol

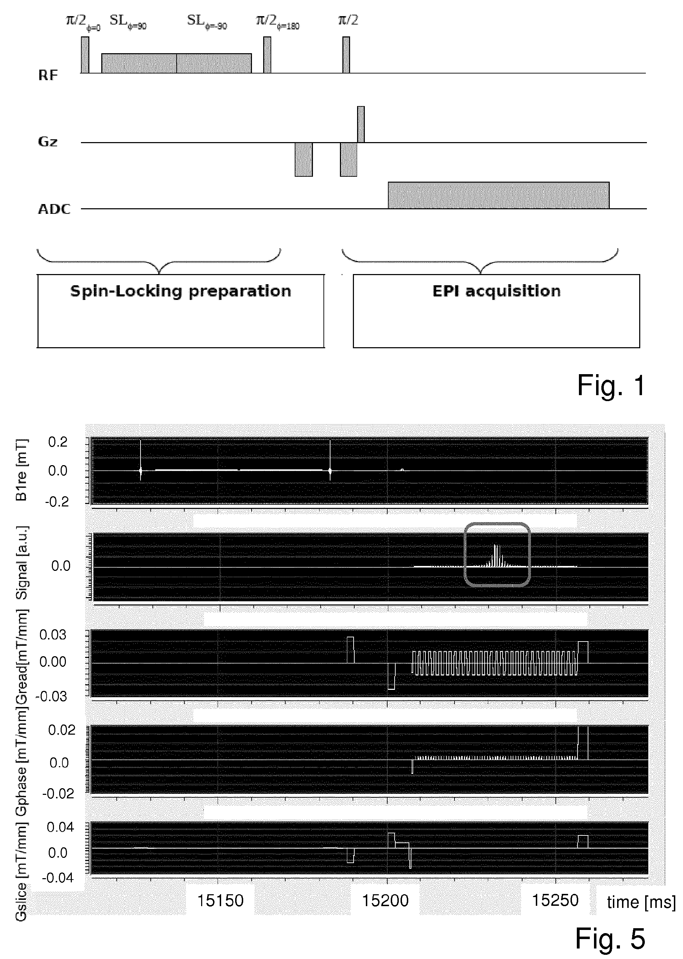

In this work we favor an approach to effectively lower the Larmor frequency down to 120 Hz in high-field MR systems based on the SL mechanism (7). Since the technique is restricted to a preselected single SL frequency for a single experiment, we preadjusted it to 120 Hz following the works of Kraus et al. and Buzsáki et al. (6,8) and the idea that pathological effects are clearly separable in the frequency domain. The basic principle of our experiment is a double irradiation rotary saturation technique. During SL, the magnetization is locked in the rotating frame by a radio frequency (RF) field, the so-called spin-lock field B_{Scanner/SL}. The effective resonance frequency in the rotating frame during the spin-lock condition is very close to ω_{Scanner/SL} = γB_{Scanner/SL}. The frequency in the rotating frame can be chosen by setting the RF power of the SL pulse. An SL-prepared echo-planar imaging (EPI) pulse sequence (10) is used (Fig. 1), extended by an on-off SL-scheme (to enable acquiring echoes with and without SL effect) and phase-cycling. The sequence starts with a nonselective π/2 pulse that excites spins that are then spin-locked in the transverse plane by the application of two phase-alternating SL pulses (+-90° phase-shifted from the phase of the first π/2 pulse). The SL pulse is split into two phase-alternating halves to reduce the effects of B1 inhomogeneity. The duration of the SL pulses is denoted as TSL. The second nonselective π/2 pulse restores the spin-locked magnetization to the longitudinal axis. All remaining transverse magnetization is spoiled by immediately following spoiler gradients. The entire SL preparation is non-selective. In our setting, the spins are sensitized to neuronal magnetic fields oscillating at the SL-frequency γB_{Scanner/SL} in the double rotating frame by suitably adjusting the amplitude and duration of the SL-pulse. If the excitation or saturation field Bₙₑᵤᵣₒ due to oscillating neuronal currents J(r,t) is on-resonant with the locked magnetization M_{SL} at frequency γB-_{Scanner/SL}, M_{SL} will be rotated away from the spin-lock axes and an excitation resp. saturation effect occurs (Fig. 2). In practice, we adopted γB_{Scanner/SL} within the frequency band of IED-related neuronal oscillations (120Hz), in order to sensitize the sequence to epileptogenic tissue. We phase cycled the initial SL-pulse between successive volumes k with phase increments of 50**k* degree in order to detect dipoles with different spatial orientation and to minimize dependencies among different scans. The non-selective SL prepared acquisition was combined with an unprepared (SL switched off) acquisition in an alternating fashion. This enables to minimize non-SL related effects during post-processing by pairwise subtraction of the signals. Overall, the required acquisition time is 13 min.

### Functional overlay

The Z-score maps (Z) were calculated on the base of the ON-OFF difference images, averaged along the temporal dimension. The Z-scores were then converted to an inverse value which expresses the degree of the resonance (saturation) effects by using the term f_{SL} = (maximum (Z)-Z). The functional plots thus show the effects with the highest f_{SL} values and the greatest cluster size. For more details see supplementary materials.

### EEG Analysis

We calculated the scalp topography of IED-related potentials by visually identifying IED in each patient's sEEG recording, isolating epochs lasting from -200ms to +200ms around the maximal negative IED deflection and averaging across epochs. We then used epoched data sets to reconstruct the most likely current sources generating the observed IED-related potentials. To this end, we applied a generic Bayesian framework that allows building and comparing source reconstruction models that incorporate prior knowledge of individual anatomy and source localization (9). Finally, we localized the SOZ by first applying a combined co-registration/segmentation procedure to preimplantation MR images and post-implantation CT scans, and then identifying the electrode positions of those channels that showed the first seizure-related signals in iEEG recordings. Details on all EEG analysis methods can be found in supplemental materials.

### Statistical Analysis

Two investigators (EA, RW) assessed independently the visual concordance of non-hemodynamic resonant saturation effects with the peak negativity of IED-related scalp potentials at the hemispheric level and the location of the SOZ at the lobar level. Confidence intervals for concordance rates were calculated using the modified Wald method (10). Cohen's kappa (κ) was calculated to test for interrater reliability of both assessments. For Bayesian comparison of source reconstruction models, we calculated the exceedance probability, i.e. our belief that a particular model is more likely, given the EEG data, than any other of the source models tested. We corrected this measure for the probability that the observed model frequencies could have arisen by chance (11). We assessed reconstruction accuracy by calculating the Euclidean distance between pc-SIRS and EEG source maxima. The Wilcoxon rank-sum test was used to compare model evidences and localization accuracies (significance level α = 0.05, corrected).

### Results

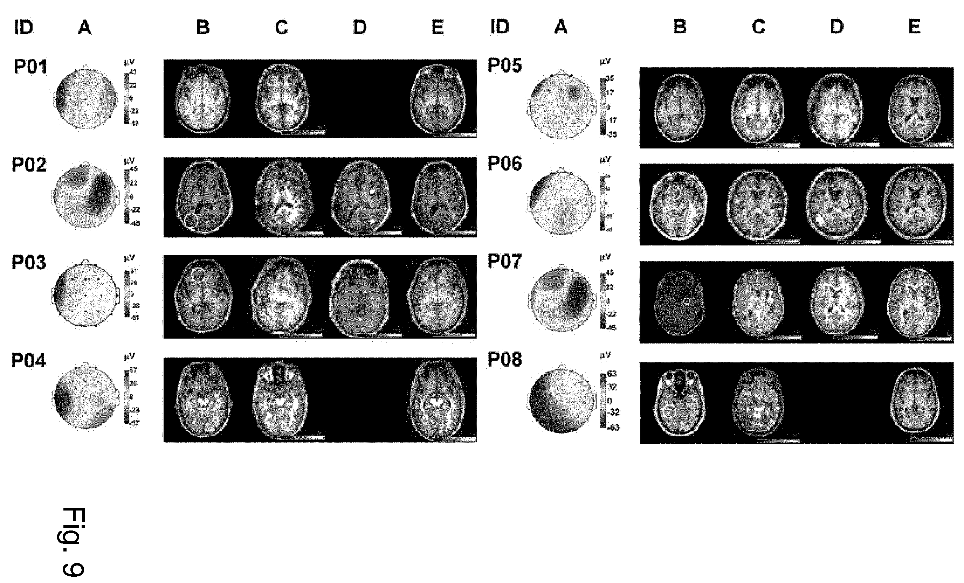

We detected focal clusters of non-hemodynamic resonant saturation (pc-SIRS) effects in z-score transformed SL-contrast images of all patients. In 6/8 patients (concordance rate and 95% CI: 0.75, 0.40 - 0.94; cases P01, P03, P04, P05, P07, P08) pc-SIRS maxima were concordant with the localization of the SOZ at the hemispheric level, and in 5/8 at the lobar level (0.63, 0.30 - 0.87) (see Fig. 9). In 2 patients with excellent post-surgical seizure control (Engel outcome class I), pc-SIRS effects were no longer detectable on follow-up imaging (P05 with lateral TLE, P07 with mesial TLE). In contradistinction, pc-SIRS effects were still present in 3 patients with less favorable post-surgical outcomes (P02 with PLE, Engel III; P03 and P06 with FLE, Engel II). P01, who had already undergone surgery before inclusion in our study, was classified as Engel IV and had still visible effects. PC-SIRS maxima were visually concordant with the peak negativity of IED-related potentials at the hemispheric level in 7/8 patients (0.88, 0.51 - 1.00). Interrater reliability for both concordance rates was high (both κ=1.,0.59-1). Including clusters containing these maxima as spatial priors in Bayesian IED-source reconstruction improved source models considerably: the protected exceedance probability of pc-SIRS-informed source models was 0.77 (unprotected 0.94), whereas the protected exceedance probability of the other two model types (side-flipped spatial priors or no priors at all) was 0.12 (0.03). Model evidence was significantly higher for informed model compared to both the side-flipped (Wilcoxon test, W=-2.38, p=0.017) and uniformed model (W=-2.24, p=0.025). Moreover, informed models found sources significantly closer (Euclidean distance mean and SD: 25±16mm) to the pc-SIRS effects than the side-flipped (76±26mm, W=0, p=0.014) or uniformed model (72±32mm,W=1, p=0.016). Figs. 3 and 4 show exemplary results in two patients. Complete results of Bayesian modeling procedures are provided in supplementary Table S 1.

### Discussion

In this study, we have demonstrated that an optimized non-hemodynamic resonant saturation technique (pc-SIRS) can measure MR-signal changes related to epileptic activity in patients with refractory focal epilepsy, which are probably closely related to neuronal current events. We detected pc-SIRS effects ipsilateral to the SOZ in 6/8 (75%) patients, and in concordance with IEDs on surface EEG in 7/8 (87.5%) patients. PC-SIRS effects resolved after epilepsy surgery only in patients who had a favorable outcome (Engel Class I), but were still present in patients with less favorable outcome (Engel Class II-IV). Furthermore, incorporating the pc-SIRS approach into an EEG source reconstruction algorithm improved the accuracy of the reconstructed sources considerably, despite of using standard clinical EEGs with low spatial resolution. Taken together, our findings suggest that pc-SIRS effects are closely linked to epileptic activity and therefore could provide additional anatomical and prognostic information in patients with focal epilepsy. Previous experiments using phase-contrast techniques and stimulation paradigms analogous to traditional BOLD-fMRI have failed to reproducibly detect neuronal currents in healthy subjects, probably due to phase cancellation effects and the weakness of stimulus-induced neuronal magnetic fields (estimated to range between 0.1-1nT) (1,3,12-14). The SIRS approach does not suffer from phase cancellation effects, and its sensitivity lies between 0.25-1nT (15-17). On the other hand, MR-signal changes related to IED are expected to be stronger than those related to signaling in the normal human brain: using a fast single-shot gradient-echo EPI sequence (TR=47ms), phase shifts of up to 1.074 radians (equivalent to 182.47nT) that occurred at a short time lag after surface IED have been reported previously (18) (*see also T1-rho contrast in the supplementary material*)*.*

This report aims primarily at a technical description of clinically applicable pc-SIRS, with some limitations to be considered: Sample size is small and postsurgical follow-up periods are relatively short; it is therefore unclear how pc-SIRS measurements generalize and whether our method is sensitive and specific enough to identify epilepsy patients de novo. Also, it remains to be investigated, whether the localization accuracy is sufficient to identify the epileptogenic zone in patients with multifocal or non-lesional epilepsies. Further prospective outcome studies that incorporate simultaneous high-density EEG-pcSIRS recordings are mandatory to confirm our results. Currently, the identification of pc-SIRS related effects is accomplished by a threshold approach. Further methodological optimization steps would include full electromagnetic wave simulations and comparisons with the measurements in order to better separate remaining artefacts and pc-SIRS related effects. In order to further enhance the detection sensitivity for clinically available 3 T MRI systems a recently proposed approach of Jiang and coworkers will be incorporated (17).

Our clinical application of pc-SIRS provides first evidence that IED-related magnitude changes of the magnetic field can be detected within a clinical routine setting in patients with refractory epilepsy. Both the phase-cycling (influence of more dipole orientations) and the approach to detect the difference between resonant and non-resonant (frequency shifted) effects may currently explain our results.

**Table 1. Clinical data of patients with drug resistant focal epilepsies**

| ID | Gender | Age / Duration (y) | Clinical Diagnosis* | Etiology | Structural MRI° | SOZ | Outcome # | Follow-up |
|---|---|---|---|---|---|---|---|---|
| P01 | F | 22 / 5 | Left LTLE | Structural (encephalitis) | None | Left superior temporal | IV (1) | no |
| P02 | M | 28 / 26 | Left PLE | Structural (ischemia) | Left temporo-parietal infarct | Left inferior parietal | III (1) | yes |
| P03 | M | 39 / 4 | Left LTLE | Structural (trauma) | Left orbitofrontal and temporal gliosis | Left orbito-frontal | II (0.5) | |
| P04 | M | 35 / 4 | Left MTLE | Structural (MTS) | Left MTS | Left mesial temporal | no surgery | no |
| P05 | F | 55 / 40 | Left LTLE | Structural (dysplasia) | Left superior temporal FCD | Left superior temporal | I (1.5) | yes |
| P06 | M | 58 / 5 | Right FLE | Structural (trauma) | Left orbitofrontal gliosis | Right frontal | II (0.5) | yes |
| P07 | F | 8 / 2 | Right MTLE | Structural (MTS) | Right MTS | Right mesial temporal | I (0.5) | yes |
| P08 | F | 42 / 35 | Left LTLE | Structural (dysplasia) | Left inferior temporal FCD | Left inferior temporal | I (0.25) | no |

| | | | | | | | | |
|---|---|---|---|---|---|---|---|---|
| Abbreviations: ID, patient identification; FCD, focal cortical dysplasia; MRI, magnetic resonance imaging; MTS, mesial temporal sclerosis; iEEG, intracranial electroencephalogram; IED, interictal epileptiform discharges; SOZ, seizure onset zone (visually identified in iEEG recordings). M/F, male or female.* Side and syndrome according to clinical information (synthesis from patient history, seizure semiology, interictal and ictal EEG). ° Side and type of potential epiletogenic lesion. # Outcome according to Engel classification (years of follow-up in brackets). | | | | | | | | |

### References

1. Bodurka J, Bandettini PA. Toward direct mapping of neuronal activity: MRI detection of ultraweak, transient magnetic field changes. Magn Reson Med. 2002;47:1052-1058http://www.ncbi.nlm.nih.gov/pubmed/12111950. Accessed August 3, 2015.
2. Petridou N, Plenz D, Silva AC, Loew M, Bodurka J, Bandettini PA. Direct magnetic resonance detection of neuronal electrical activity. Proc Natl Acad Sci USA. 2006;103:16015-16020http://www.pubmedcentral.nih.gov/articlerender.fcgi?artid=1635119&tool=pmcentr ez&rendertype=abstract. Accessed August 12, 2015.
3. Xiong J, Fox PT, Gao J-H. Directly mapping magnetic field effects of neuronal activity by magnetic resonance imaging. Hum Brain Mapp. 2003;20:41-49http://www.ncbi.nlm.nih.gov/pubmed/12953305. Accessed July 25, 2015.
4. Halpern-Manners NW, Bajaj VS, Teisseyre TZ, Pines A. Magnetic resonance imaging of oscillating electrical currents. Proc Natl Acad Sci USA. 2010;107:8519-8524.
5. Witzel T, Rosen BR, Lin F-H, Wald LL. Stimulus Incuded Rotary Saturation (SIRS); a new method for the direct detection of neuronal currents with MRI T. 2007;1809:2007.
6. Kraus RH, Volegov P, Matlachov a., Espy M. Toward direct neural current imaging by resonant mechanisms at ultra-low field. Neuroimage. 2008;39:310-317.
7. Abragam A. The Principles of Nuclear Magnetism. 1961.https://books.google.de/books/about/The_principles_of_nuclear_magnetism.html?id= 9M8U_JK7K54C&pgis=1. Accessed October 26, 2015.
8. Buzsáki G, Silva FL Da. High frequency oscillations in the intact brain. Prog Neurobiol. 2012;98:241-249.
9. Litvak V, Mattout J, Kiebel S, et al. EEG and MEG data analysis in SPM8. Comput Intell Neurosci. 2011;2011:852961http://www.pubmedcentral.nih.gov/articlerender.fcgi?artid=3061292&t ool=pmcentrez&rendertype=abstract. Accessed August 17, 2015.
10. Agresti A, Coull BA. Approximate is Better than "Exact" for Interval Estimation of Binomial Proportions. Am Stat. Taylor & Francis Group; 1998;52:119-126http://www.tandfonline.com/doi/abs/10.1080/00031305.1998.10480550. Accessed August 19, 2015.
11. Rigoux L, Stephan KE, Friston KJ, Daunizeau J. Bayesian model selection for group studies - revisited. Neuroimage. 2014;84:971-985http://www.ncbi.nlm.nih.gov/pubmed/24018303. Accessed August 14, 2015.
12. Chu R, de Zwart JA, van Gelderen P, et al. Hunting for neuronal currents: absence of rapid MRI signal changes during visual-evoked response. Neuroimage. 2004;23:1059-1067http://www.ncbi.nlm.nih.gov/pubmed/15528106. Accessed August 12, 2015.
13. Parkes LM, de Lange FP, Fries P, Toni I, Norris DG. Inability to directly detect magnetic field changes associated with neuronal activity. Magn Reson Med. 2007;57:411-416http://www.ncbi.nlm.nih.gov/pubmed/17260380. Accessed August 12, 2015.
14. Tang L, Avison MJ, Gatenby JC, Gore JC. Failure to direct detect magnetic field dephasing corresponding to ERP generation. Magn Reson Imaging. 2008;26:484-489http://www.pubmedcentral.nih.gov/articlerender.fcgi?artid=2459244&tool=pmcentrez &rendertype=abstract. Accessed October 26, 2015.
15. Witzel T, Lin F-H, Rosen BR, Wald LL. Stimulus-induced Rotary Saturation (SIRS): a potential method for the detection of neuronal currents with MRI. Neuroimage. Elsevier; 2008;42:1357-1365http://www.deepdyve.com/lp/elsevier/stimulus-induced-rotary-saturation-sirs-a-potential-method-for-the-QqEgEoi2Y7. Accessed August 12, 2015.
16. Halpern-Manners NW, Bajaj VS, Teisseyre TZ, Pines A. Magnetic resonance imaging of oscillating electrical currents. Proc Natl Acad Sci USA. 2010;107:8519-8524.
17. Jiang X, Sheng J, Li H, et al. Detection of subnanotesla oscillatory magnetic fields using MRI. Magn Reson Med. 2015;00:n/a - n/ahttp://doi.wiley.com/10.1002/mrm.25553.
18. Sundaram P, Wells WM, Mulkern R V, et al. Fast human brain magnetic resonance responses associated with epileptiform spikes. Magn Reson Med. 2010;64:1728-1738http://www.pubmedcentral.nih.gov/articlerender.fcgi?artid=3681097&tool=pmcentrez &rendertype=abstract. Accessed October 26, 2015.

### Supplementary Information

### Spin-Locking: General prirtciple

Usual MR techniques use radio-frequency (rf) pulses tuned to the resonance frequency of water protons, e.g. 128MHz at 3 Tesla. The lower the field, the lower this frequency. In order to generate ultra-low fields, e.g. 120Hz you need either a scanner with the capability to drive the B0 field down or you use a technique which allows to use two resonant frequencies: 128MHz *and* 120Hz (double rotating frame). This is accomplished by the spin-lock and doubly irradiation rotary saturation technique. With this approach the spins are excited (influenced) by a typical MR rf-pulse at the resonance (carrier) frequency of 128MHz and - depending on its amplitude, duration and the axis of irradiation - this so-called spin-locking rf-pulse is able to force the magnetization to precess around its axis at a much lower (additional) frequency of e.g. 120Hz. If there is another oscillating pulse Bᵣₒₜ (the oscillating neuronal field) present, which is in resonance with the 120Hz, then the spins are excited by this Bᵣₒₜ pulse which results in a pre-saturation effect (see Fig.2) that scales with the spectral power and amplitude of the oscillating neuronal field.

### Relation of neuronal activity and pc-SIRS effects

The current density J(r) produced by neuronal activity can be described by two additive components: a primary J^{P} and a volume current density J^{V}: J(r,t) = J^{P}(r,t) + σ(r)E(r,t) (primary current density J^{P}(r), volume/return current density J^{V}(r,t) = σ(r)E(r,t), electric field strength E, conductivity σ). The primary active component J^{P} is mainly inside or in the vicinity of a cell, whereas the volume part J^{V} is passive and the result of the macroscopic electric field on charge carriers in the conducting medium. PC-SIRS seems to be sensitive to both, J^{V} and J^{P}. The relation of the current density with the magnetic field density Bₙₑᵤᵣₒ is derived from Biot-Savart Bₙₑᵤᵣₒ(r,t) = (µo/4π) ∫ J(r',t)x R/R³ dv' - therefore there is no simple correlation between the EEG-amplitudes and the pc-SIRS z-scores to be expected. For the pc-SIRS effects to be measurable by means of magnetic resonance it is necessary that the condition of a collinear orientation of the direction of B_{Scanner/SL} and an orthogonal orientation of Bₙₑᵤᵣₒ relative to the spin-locking axes is fulfilled for the whole brain. This is to some degree fulfilled by changing the phase of the SL-pulse in steps of 50° (phase-cycling).

### T1-rho contract

As shown by Witzel et al. (S1) in healthy tissue the changes of magnetization in case of resonant rotary saturation is in the order of 0.1% for a 100ms spin-locking and a rotary field Brot amplitude of 6.8nT and around 0.01% (and less) for a 1nT field (the spin-locked magnetization is rotated away from the spin-lock-field). This resonant effect depends on the spectral power and amplitude of the oscillating neuronal currents (Brot) which should be significantly higher in epileptic zones (factor ∼100). Therefore we expect that in all other cases where the spin-locked magnetization simply returns to the z-axis for subsequent imaging, just slightly modified by T1rho, TSL, the spin-lock field amplitude, the temperature and the tissue type, the change should be significantly lower. According to the results in the work of Witzel et al. and our results this ratio between the changes of magnetization within epileptic zones and healthy tissue is at least of the order 0.1/0.01=10 and even higher and thus the pc-SIRS contrast is inherently separated from the T1-rho effects (no special post-processing was necessary).

### Functional overlay

In order to localize these effects neuro-anatomically, overlays of the fSL-maps with coregistered T1 weighted images (SPM8: http://www.fil.ion.ucl.ac.uk/spm/) were generated within the frame of the MRIcro software (http://www.mccauslandcenter.sc.edu/mricro/). The fSL-maps are weighted by a factor of 1000. In MRIcro the options *"Overlaylload functional"* overlay and "*Minimum cluster size"* (#voxels) were used.

### Sequence-related effects

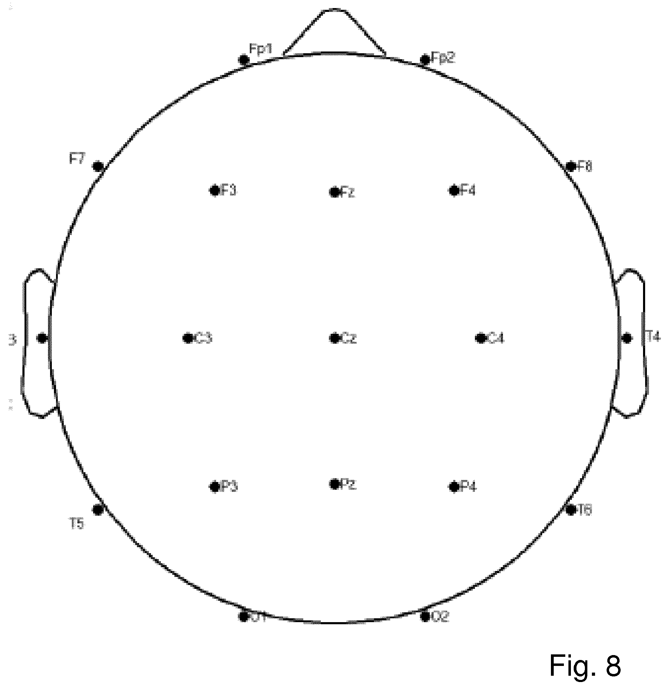

The simulation of the entire sequence code (see Charagundla et al. (S2)) indicated that essential pc-SIRS effects are not generated by stimulated or indirect echoes (Fig. 5). The effects of B1 inhomogeneity were more pronounced for TSL = 50ms compared to TSL = 100ms (Fig. S2), thus the self compensating cancellation effect seems to be more effective for longer TSL. Remaining artifacts (Fig. 8) are due to off-resonance (chemical shifts or B0 inhomogeneities) spin-locking effects and SAR related limitations of TSL and therefore non-perfect self-compensating cancellation. In order to optimally detect the artefact-free effects and to meet the SAR constraints, we used TSL= 100ms pulses to acquire patient data.

### EEG Recordings

Scalp EEG recordings were acquired using chlorided silver cup electrodes in a standard clinical 10/20 montage, including 2-channel electrocardiography, electromyography and electrooculography channels and additional sphenoidal and mastoid electrodes (S3). Intracranial EEG signals were recorded with intracranial strip-, and depth-electrodes (AD-TECH, Racine, WI, U.S.A). We used an extracranial reference electrode located between positions Fz and Pz in the 10/20 system. For strip and depth electrodes the contacts were numbered starting at the tip of the electrodes. A NicoletOne recording system with a C64 amplifier (VIASYS Healthcare, Inc., Madison, WI, U.S.A.) was employed. Scalp EEG data were recorded with a sampling rate of 256Hz, intracranial data with a sampling rate of 1024Hz.

### EEG reprocessing

Scalp EEG preprocessing and analysis was performed using EEGLAB 13.0.1b (http://sccn.ucsd.edu/eeglab/)for MATLAB (MathWorks, Natick, MA, U.S.A.). From the individual clinical electrode montages, nine-teen consistent channels across all subjects were kept (see Fig. 8). Artifacts were detected and removed from the raw EEG traces using independent component analysis (extended infomax algorithm with default settings) (S4). All channels were then re-referenced to the common signal average and band pass filtered between 1 and 30Hz using a finite-impulse response filter.

### EEG Analysis

### 1. Topography of interictal epileptiform discharges

Interictal epileptiform discharges (IED) were visually identified by one neurologist trained in clinical EEG analysis (EA). IED were marked as zero-duration events centered on the first maximal negative deflection of each IED, and brief epochs from -200 ms to +200 ms around each event were extracted and averaged (S5). IED voltage topographies were then plotted using EEGLAB functions and visually compared to the distribution of pc-SIRS effects.

### 2. Identification of seizure onset zone

The seizure onset zone was determined by visual analysis by a senior epileptologist (KS). To identify the intracranial location of each electrode, postimplantation CT and preimplantation T1- weighted MR images were fused using a multimodal coregistration procedure using SPM8 (http://www.fil.ion.ucl.ac.uk/spm/software/spm8/) for MATLAB (MathWorks, Natick, MA, U.S.A.). CT scans and MRI images were coregistered by maximizing the normalized mutual information (S6). MR-images were then segmented into grey matter (GM), white matter (WM), cerebrospinal fluid (CSF) and non-brain tissue probability maps using SPM8's unified segmentation framework (S7). GM, WM and CSF masks were then binarized to retain voxels with 80% probability for each tissue class and recombined to form a brain mask that was applied to the coregistered CT image to extract in-brain voxels. Electrodes were then identified as high-density artifacts with Hounsfield units >3000 and projected onto the MR images. Channel assignments to each electrode position were identified using surgical implantation protocols.

### 3. Bayesian source recortstructiort.

The problem of estimating then intra-cranial distribution of current sources, based on surface EEG signals alone, is mathematically ill-posed, since an infinite number of source configurations could generate a particular surface recording (S8). In order to converge to an optimal solution, source reconstruction algorithms need to incorporate prior assumptions on how the surface signals were generated (S8). We hyopthesized that pc-SIRS effects do indeed represent a volumetric measurement of neuronal currents, and that incorporating them into reconstruction algorithms would therfore increase the plausibilty (or evidence) of the recovered source configurations.

To test this hypothesis, we caculated the EEG sources of interictal epileptiform discharges (IED) using a Bayesian analysis framework available in SPM12 (http://www.fil.ion.ucl.ac.uk/spm/software/spm12/). This framework is a generic probabilistic approach to distributed linear (DL) source reconstruction models, an established methodology in EEG source analysis (S9). DL source models assume that observed scalp signals are a linear function of the dipolar current sources distributed across the brain, where a so-called lead field matrix encodes how much a given surface signal varies with source activity. The Bayesian formulation of DL models allows the priniciple incorporation of individual cortical anatomy (via segmentation of T1-weighted images) and other prior information into the source reconstruction (S10). Details on the mathematical background are summarized in López et la. (S11), and in Wipf and Nagarjan (S12)and a practical step-by-step introduction is given in Litvak et al. (S13).

For our implementation, we used the same T1-weighted MRI scans used for CT coregistration to calculate individual cortical meshes with n=8196 vertices, where each of the vertices is assumed to represent a current dipole. We next used the boundary elements model to calculate the lead field matrix that encodes the effect of each vertex on the surface channels (S14). For model inversion, we used the multiple sparse priors approach to model source covariances, which leads to source reconstructions with good localization accuarcy (S11, S15). We modelled source activity across the whole IED event, i.e. from -200 ms to +200 ms after peak negativity, since we could not assume correspondence between pc-SIRS effects and specific IED components. To introduce priors on source localization (i.e. to weight groups of cortical dipoles) we included for each subject the binarized cluster that contained the pc-SIRS maximum ("informed model"). Note that this is a soft constraint that does not force source estimates to lie within this cluster (S11). As a control, we mirrored this cluster on the brain's midline, thus deliberately building a invalid source reconstruction model. This model was introduced as a "control" model for the anatomical specificity of its "informed" counterpart. Finally, we reconstructed source activity without any prior information (baseline or "zero" model). Model comparison at the group level was done using log model evidences. We first calculated the exceedance probability, which quantifies our belief that a particular model is more likely than any other, given the data and the competing models. We corrected this value for the probability that the observed model distribution could have arisen by chance (S16). Images of the source reconstructions and pc-SIRS clusters were projected into MNI space, and the Euclidean distance between the pc-SIRS peak and the source peak was calculated.

Results are summarized in Table S1 and Fig. 9 below. Across our pilot series, the informed model was far more probable than the control or zero model (exceedance probablity 0.77 versus 0.12). The source peaks from the informed model had on average a smaller distance to the pc-SIRS cluster peak than the control model (paired T-test, *t*(7) = -8.89, p<.001) and the zero model (paired T-test, *t*(7) = -4.64, p=.002). Distributional assumptions where checked with QQ-plots and the Kolmogorv-Smirnov statistic D for all distances before calculating T-tests. There was no deviation from normality (D for informed model distances: .58, p>.5, for control model distances: .57, p>.5, for zero model distances: .42, p>.5).

**Table S1. Results of Bayesian model selection of EEG source reconstruction models with or without pc-SIRS priors**

| | | **pc-SIRS cluster** | | | | **Model 1: With pc-SIRS cluster prior** | | | | | | **Model 2: Mirrored pc-SIRS cluster prior** | | | | | | **Model 3: No pc-SIRS prior** | | | | | |
|---|---|---|---|---|---|---|---|---|---|---|---|---|---|---|---|---|---|---|---|---|---|---|---|
| **ID** | **IED** | **Size (voxets)** | **Cluster peak** | | | **Source peak** | | | **d** | **%Var** | **logE** | **Source peak** | | | **d** | **%Var** | **logE** | **Source peak** | | | **d** | **%Var** | **logE** |
| | | | x | *y* | z | x | *y* | z | | | | x | *y* | z | | | | *x* | y | z | | | |
| P01 | 11 | 94 | -43 | -42 | 1 | -43 | -35 | -6 | 10 | 43.1 | 188.6 | 35 | -40 | 0 | 78 | 24.6 | 129.7 | 6 | -67 | 36 | 65 | 41.0 | 184.3 |
| P02 | 6 | 100 | 61 | -11 | 0 | 62 | -13 | 4 | 5 | 97.5 | 666.0 | 43 | -35 | -3 | 30 | 97.7 | 643.4 | 12 | -73 | 28 | 84 | 97.4 | 633.2 |
| P03 | 15 | 1214 | -49 | -59 | -2 | -49 | -29 | -3 | 30 | 97.9 | 3291.9 | -2 | 4 | 11 | 80 | 97.6 | 3265.8 | -40 | -76 | 3 | 20 | 98.1 | 3232.3 |
| P04 | 20 | 85 | -26 | -41 | -3 | -39 | -81 | 9 | 44 | 97.8 | 5274.6 | 36 | 40 | 29 | 107 | 97.2 | 5233.7 | 36 | 40 | 30 | 107 | 97.7 | 5197.5 |
| P05 | 13 | 97 | -59 | -18 | -1 | -45 | 5 | 29 | 40 | 81.5 | 1066.5 | 45 | 5 | 29 | 111 | 53.4 | 879.4 | 45 | 5 | 29 | 111 | 80.2 | 1073.0 |
| P06 | 8 | 54 | 39 | -8 | 12 | 39 | -14 | 11 | 7 | 90.5 | 1788.2 | 28 | 47 | 14 | 56 | 80.2 | 1704.1 | 42 | 28 | 30 | 40 | 79.5 | 1600.4 |
| P07 | 2 | 1274 | 42 | -10 | 4 | 35 | -35 | 3 | 26 | 95.7 | -268.2 | 3 | -56 | 49 | 75 | 96.0 | -260.2 | 28 | -65 | -2 | 57 | 96.3 | -269.6 |
| P08 | 13 | 598 | -62 | -66 | 5 | -57 | -27 | 14 | 40 | 97.9 | 2863.9 | 1 | -33 | 23 | 73 | 97.2 | 2783.1 | 1 | -3 | 14 | 90 | 97.9 | 1332.8 |
| M | 12 | 98.5 | | | | | | | 28 | 96.6 | 1427.4 | | | | 77 | 96.6 | 1291.8 | | | | 75 | 96.9 | 1202.9 |
| R | 1,20 | 54,1274 | | | | | | | 5,44 | 43.1,97.9 | -268.2,5274.6 | | | | 30,111 | 24.8,97.7 | -260.2,5433.7 | | | | 20,111 | 41.0,98.1 | -269.6,5197.5 |
| µ | 10.9 | 439.5 | | | | | | | 25 | 87.7 | 1658.9 | | | | 76 | 80.5 | 1797.4 | | | | 72 | 86.0 | 1623.0 |
| o | 5.8 | 527.5 | | | | | | | 16 | 18.9 | 1855.5 | | | | 26 | 27.3 | 1856.8 | | | | 32 | 19.9 | 1788.3 |
| XP | | | | | | | | | | | 0.94 | | | | | | 0.03 | | | | | | 0.03 |
| PXP | | | | | | | | | | | 0.77 | | | | | | 0.12 | | | | | | 0.12 |

| | | | | | | | | | | | | | | | | | | | | | | | |
|---|---|---|---|---|---|---|---|---|---|---|---|---|---|---|---|---|---|---|---|---|---|---|---|
| Abbreviations: IED. interictal epileptiform discharges (total number); MR- median range; µ(o-mean/standard deviation, NCL neuronal current imaging: d Euclidean distance (mm) from source peak to pc-SIRS cluster peak, %Var. percent variance explained; logE, log-model evidence; XP, exceedance probability; PXP protected XP Coordinates (x,y,z) are m MNI stereotaxic space (mm). | | | | | | | | | | | | | | | | | | | | | | | |

### Supplementary Reference

S1. Witzel T, Lin F-H, Rosen BR, Wald LL. Stimulus-induced Rotary Saturation (SIRS): A potential method for the detection of neuronal currents with MRI. NeuroImage. 2008;42(4):1357-65.
S2. Charagundla SR, Borthakur A, Leigh JS, Reddy R. Artifacts in T(1rho)-weighted imaging: correction with a self-compensating spin-locking pulse. Journal of magnetic resonance (San Diego, Calif: 1997).2003;162(1):113-21.
S3. Klem GH, Luders HO, Jasper HH, Elger C. The ten-twenty electrode system of the International Federation. The International Federation of Clinical Neurophysiology. Electroencephalography and clinical neurophysiology Supplement. 1999;52:3-6.
S4. Delorme A, Sejnowski T, Makeig S. Enhanced detection of artifacts in EEG data using higher-order statistics and independent component analysis. Neuroimage. 2007;34(4):1443-9.
S5. Murray MM, Brunet D, Michel CM. Topographic ERP analyses: a step-by-step tutorial review. Brain topography. 2008;20(4):249-64.
S6. Ashburner J, Friston K. Multimodal image coregistration and partitioning--a unified framework. Neuroimage. 1997;6(3):209-17.
S7. Ashburner J, Friston KJ. Unified segmentation. Neuroimage. 2005;26(3):839-51.
S8. Michel CM, Murray MM, Lantz G, Gonzalez S, Spinelli L, Grave de Peralta R. EEG source imaging. Clinical neurophysiology : official journal of the International Federation of Clinical Neurophysiology. 2004;115(10):2195-222.
S9. Murphy TH, Corbett D. Plasticity during stroke recovery: from synapse to behaviour. Nat Rev Neurosci. 2009;10(12):861-72.
S10. Henson RN, Flandin G, Friston KJ, Mattout J. A parametric empirical Bayesian framework for fMRI-constrained MEG/EEG source reconstruction. Hum Brain Mapp. 2010;31(10):1512-31.
S11. Lopez JD, Litvak V, Espinosa JJ, Friston K, Barnes GR. Algorithmic procedures for Bayesian MEG/EEG source reconstruction in SPM. Neuroimage. 2014;84:476-87.
S12. Wipf D, Nagarajan S. A unified Bayesian framework for MEG/EEG source imaging. Neuroimage. 2009;44(3):947-66.
S13. Litvak V, Mattout J, Kiebel S, et al. EEG and MEG data analysis in SPM8. Computational intelligence and neuroscience. 2011;2011:852961.
S14. Hamalainen MS, Sarvas J. Realistic conductivity geometry model of the human head for interpretation of neuromagnetic data. IEEE transactions on bio-medical engineering. 1989;36(2):165-71.
S15. Friston K, Harrison L, Daunizeau J, et al. Multiple sparse priors for the M/EEG inverse problem. Neuroimage. 2008;39(3):1104-20.
S16. Rigoux L, Stephan KE, Friston KJ, Daunizeau J. Bayesian model selection for group studies - revisited. Neuroimage. 2014;84:971-85.

## Claims

1. A magnetic resonance (MR) imaging method for detecting neuronal activity in a subject's brain positioned in a ¹H-MR imaging system having a static magnetic field in z-direction, the method comprising:
a) performing a first MR imaging sequence part with a MR imaging system having a static magnetic field in z-direction, said first sequence part comprising:
- a preparatory spin-locking pulse sequence part comprising an initial spin-tipping pulse followed by a spin-lock pulse followed by a final spin-tipping pulse, followed by spoiler gradient pulses for removing any remaining transverse magnetization;
wherein said initial spin-tipping pulse is a 90° pulse with an initial phase ϕ_{1SL} in the rotating system and wherein said final spin-tipping pulse is a 90° pulse with a final phase ϕ_{1SL} + 180°;
wherein said spin-lock pulse consists of a rectangular pulse having an amplitude B_{1lock} and a spin-lock pulse duration TSL and consisting of a first pulse portion with a first phase ϕ_{1SL} + 90° followed by a second pulse portion with a second phase ϕ_{1SL} - 90° or, vice versa, consisting of a first pulse portion with a first phase ϕ_{1SL} - 90° followed by a second pulse portion with a second phase ϕ_{1SL} + 90°, said spin-lock pulse having an amplitude B_{1lock} corresponding to a ¹H-Larmor frequency of 100 to 200 Hz;
- an acquisition pulse sequence part to acquire first MR data, wherein said acquisition pulse sequence part is an echo planar imaging sequence;
said step a) being followed or preceded by step b)
b) performing a second MR imaging sequence part with said MR imaging system, said second sequence part consisting of said acquisition pulse sequence part to acquire second MR data;
c) repeating steps a) and b) for a predetermined number of instances, wherein for each instance the initial spin-lock phase ϕ_{1SL} is increased, with respect to the preceding instance, by a predetermined phase increment Δϕ_{1SL}, thereby obtaining multiple sets of first and second MR data;
d) averaging said first MR data and said second MR data to obtain averaged first MR data and averaged second MR data, respectively;
e) obtaining difference MR data by subtracting said averaged second MR data from said averaged first MR data; and
f) generating at least one slice image from said difference MR data, wherein zones of comparatively high brightness are indicative of fast oscillating neuronal currents with a frequency in the range of 100 to 200 Hz.

2. The method according to claim 1, wherein said echo planar imaging sequence is a gradient echo echo planar imaging (GE-EPI) sequence.

3. The method according to claim 1 or 2, wherein the spin-lock pulse duration TSL is 50 to 200 ms, preferably about 100 ms.

4. The method according to one of claims 1 to 3, wherein the phase increment Δϕ_{1SL} is 40° to 60°, preferably about 50°.

5. The method according to one of claims 1 to 4, wherein said image is represented as a z-score map obtained by a raw image reconstructed from said difference MR data.

6. The method according to claim 5, wherein said z-score map includes a threshold of 50 to 70%, preferably about 60%.

7. A computer program product comprising program code means stored on a computer readable medium for causing a computer system to control the execution of the method according to any one of claims 1 to 6 when the computer program product is run on the computer system.
